# FASCICULE DE BREVET EUROPEEN

(11) **EP 1 009 458 B1**
(45) Date de publication et mention de la délivrance du brevet: **28.04.2004**
(21) Numéro de dépôt: 98903081.2
(22) Date de dépôt: 19.01.1998
(51) Int. Cl.: A61M 11/00, A61F 9/00, B05B 15/08

(54) **DISPOSITIF POUR DISTRIBUER UN PRODUIT FLUIDE OU PULVERULENT A UNE DISTANCE PREDETERMINEE**
VORRICHTUNG ZUR ABGABE EINES FLÜSSIGEN ODER PULVERFÖRMIGEN PRODUKTS IN EINEM GEWISSEN ABSTAND
DEVICE FOR DISPENSING A FLUID OR POWDERY PRODUCT AT A PREDETERMINED DISTANCE

(30) Priorité: 20.01.1997 FR 9700541
(43) Date de publication de la demande: 21.06.2000
(73) Titulaire: VALOIS S.A.S., 27110 Le Neubourg (FR); SANOFI-SYNTHELABO, 75013 Paris (FR)
(72) Inventeur: ARGHYRIS, Laurent, F-76300 Sotteville-lès-Rouen (FR); BEDOS, Michel, F-34380 Viols-le-Fort (FR)
(74) Mandataire: CAPRI
(86) Numéro de dépôt international: PCT/FR1998/000087
(87) Numéro de publication internationale: WO 1998/031412

(56) Documents cités:
- FR-A- 2 682 305
- GB-A- 2 000 555
- US-A- 4 296 317
- US-A- 5 588 564

## Description

La présente invention concerne une dispositif de distribution de produit fluide ou pulvérulent et plus particulièrement un tel dispositif muni d'un moyen permettant l'utilisation à une distance déterminée de l'endroit à traiter.

Un dispositif de pulvérisation ou de distribution d'une substance fluide à actionnement mécanique est connu du document FR-2 682 305. Dans ce dispositif, une pompe est armée au moyen d'un ressort d'actionnement, le dispositif comprenant des moyens de verrouillage du ressort, des moyens de déclenchement, et un capot de protection de boîtier doté de moyens de réarmement du dispositif qui deviennent opérants lors de la fermeture dudit capot.

Ce dispositif antérieur fonctionne de manière satisfaisante notamment en garantissant une reproductibilité de la dose du fait de son déclenchement indépendant de l'utilisateur, mais ne permet pas de remplir certaines exigences pouvant se poser en fonction du type de produit distribué et du type d'utilisation du dispositif.

Ainsi, pour certains produits, par exemple pour des produits à usage systémique tels que des thérapies hormonales substitutives, la surface à pulvériser doit être définie de la manière la plus précise possible afin de reproduire la biodisponibilité. En particulier, dans le cas d'une utilisation transdermale, il est important de répartir le liquide pulvérisé sur une surface homogène prédéterminée et reproductible pour en favoriser l'absorption reproductible par la peau.

D'autre part, dans le cas d'utilisation ophtalmologique, il est important, pour favoriser l'observance, de faire en sorte que le produit, par exemple la goutte de liquide, atteigne l'oeil avec une même cinétique.

En outre, dans le cas d'utilisation pulmonaire, il est parfois important, pour réduire les effets secondaires, de faire en sorte que seules les particules pulvérisées de petite taille soient aspirées par le patient, et d'éviter que les grosses particules, qui ne peuvent être inhalées du fait de leur taille, se déposent dans la bouche du patient, pouvant générer des effets indésirables, tel que la candidose, par exemple. Pour ce faire, il faudrait pulvériser à une distance suffisamment grande pour que les grandes particules lourdes n'atteignent pas la bouche, mais suffisamment petite pour que les petites particules soient aspirées.

La présente invention a pour but de fournir un dispositif de distribution de produit qui satisfasse aux exigences précitées et qui ne présente pas les inconvénients susmentionnés. En particulier, la présente invention a pour but de fournir un dispositif de distribution de produit avec lequel l'efficacité et/ou le bénéfice thérapeutique du produit distribué est maximal(e).

La présente invention a aussi pour but de fournir un dispositif de distribution de produit qui garantisse simultanément la reproductibilité de la dose, la reproductibilité de la surface à traiter et la reproductibilité de la cinétique du produit dosé, c'est-à-dire qui garantisse la reproductibilité de l'action et de l'efficacité du produit sur le patient.

La présente invention a encore pour but de fournir un tel dispositif qui permette de favoriser l'observance et/ou d'optimiser la biodisponibilité et/ou de réduire les effets secondaires.

La présente invention a aussi pour but de fournir un tel dispositif qui soit simple à réaliser et à utiliser, et qui notamment assure à l'utilisateur une efficacité maximale à chaque actionnement.

La présente invention a donc pour objet un dispositif de pulvérisation ou de distribution d'un produit fluide ou pulvérulent sur une surface à traiter et/ou un organe d'entrée, comportant :
- un organe de distribution, comportant un orifice de distribution, ledit organe de distribution étant associé à un réservoir contenant ledit produit et étant mobile entre une position de repos et une position de distribution, et
- des moyens d'actionnement pour déplacer ledit organe de distribution vers sa position de distribution pour délivrer une dose de produit,
caractérisé en ce que le dispositif comporte en outre
- des moyens jauge de distance déplaçables entre une position de repos, dans laquelle ils empêchent l'actionnement dudit organe de distribution , et une position d'utilisation, dans laquelle lesdits moyens jauge de distance s'étendent dans une direction parallèle à la direction de distribution du produit fluide ou pulvérulent, de telle sorte que les moyens jauge de distance peuvent s'appuyer sur et/ou autour de la surface à traiter et/ou de l'organe d'entrée, de telle sorte que l'orifice de distribution est situé à une distance fixe prédéterminée de ladite surface à traiter et/ou dudit organe d'entrée, le déplacement desdits moyens jauge de distance de leur position d'utilisation vers leur position de repos, après actionnement de l'organe de distribution, ramenant ledit organe de distribution de sa position de distribution vers sa position de repos.

De préférence, lesdits moyens d'actionnement comportent des moyens élastiques, agissant sur l'organe de distribution, lorsqu'il est en position de repos, pour le solliciter vers une position de distribution où il délivre une dose complète de produit, des moyens de verrouillage desdits moyens élastiques pour maintenir l'organe de distribution dans sa position de repos et des moyens de déclenchement actionnables par l'utilisateur pour libérer lesdits moyens de verrouillage et ainsi amener l'organe de distribution vers sa position de distribution. Ainsi, le déclenchement et la distribution de la dose sont indépendants de l'effort d'actionnement et la reproductibilité de la dose est garantie, même si l'utilisateur est très faible.

Avantageusement, lesdits moyens jauge de distance comportent des moyens de réarmement qui, lorsque les moyens jauge de distance sont ramenés de leur position d'utilisation vers leur position de repos, agissent sur l'organe de distribution pour le ramener vers sa propre position de repos, contre la force des moyens élastiques, après quoi ces moyens élastiques sont verrouillés par lesdits moyens de verrouillage.

Avantageusement, lesdits moyens jauge de distance sont réalisés sous la forme d'un fourreau comportant un capot, ledit capot, obturant l'orifice de distribution de l'organe de distribution et ledit fourreau recouvrant lesdits moyens de déclenchement, empêchant ainsi un actionnement du dispositif, dans la position de repos desdits moyens jauge de distance. Le dispositif de l'invention permet donc en outre de protéger l'orifice de distribution pendant le stockage, par exemple contre la poussière, et d'empêcher un actionnement non souhaité du dispositif.

De préférence, lesdits moyens jauge de distance sont en permanence reliés audit dispositif, le déplacement desdits moyens jauge de distance entre la position de repos et leur position d'utilisation étant constitué d'une translation parallèle à l'axe central du dispositif et d'une rotation autour d'un axe perpendiculaire à l'axe central du dispositif, lesdits moyens de réarmement agissant pendant ledit mouvement de translation lorsque les moyens jauge de distance sont ramenés vers leur position de repos.

Avantageusement, lesdits moyens jauge de distance comportent deux pattes reliées au corps du dispositif, lesdites pattes coulissant dans deux rainures ménagées dans ledit corps, lesdites deux pattes pouvant en outre pivoter d'environ 90° autour d'un axe de rotation disposé transversalement à l'axe central du dispositif au niveau des extrémités supérieures des deux rainures.

De préférence, le dispositif comporte en amont dudit orifice de distribution, un gicleur permettant de définir la configuration spatiale de la pulvérisation de la dose de produit. Ceci permet encore davantage de prédéterminer la surface à traiter et de la reproduire exactement à chaque actionnement.

Avantageusement, les moyens jauge de distance sont adaptés à coopérer avec une partie du corps humain.

Avantageusement, l'organe de distribution est une pompe.

Avantageusement, l'organe de distribution est une valve doseuse.

Avantageusement, les moyens élastiques sont réalisés sous la forme d'un ressort.

Selon un mode de réalisation avantageux, le dispositif de pulvérisation ou de distribution est un distributeur de poudre, tel qu'un inhalateur actif ou passif. Bien entendu, tout autre type quelconque de distributeur de poudre est envisageable.

Selon une forme de réalisation avantageuse de l'invention, lesdits moyens jauge de distance sont adaptés à s'appuyer sur le corps du patient, à proximité de ladite surface à traiter, le produit agissant de manière transdermale et/ou systémique sur ladite surface à traiter.

Avantageusement, le produit comporte des hormones substitutives.

Avantageusement, le produit comporte des oestrogènes.

Selon une autre forme de réalisation avantageuse de l'invention, le produit est un produit liquide, lesdits moyens jauge de distance étant adaptés à s'appuyer sous ou autour d'un oeil.

Selon encore une autre forme de réalisation avantageuse de l'invention, lesdits moyens jauge de distance sont adaptés à s'appuyer sur ou autour de la bouche de l'utilisateur, le produit étant distribué à l'intérieur desdits moyens jauge de distance, la longueur desdits moyens jauge de distance étant choisie de telle sorte que seules les particules de produit finement pulvérisées atteignent la bouche, les particules plus grosses tombant sous l'effet de leur poids dans lesdits moyens jauge de distance avant d'atteindre la bouche.

D'autres caractéristiques et avantages apparaîtront au cours de la description détaillée suivante d'un mode de réalisation de l'invention, donné à titre d'exemple non limitatif, en regard des dessins joints, sur lesquels :
- la figure 1 est une vue schématique en coupe d'un mode de réalisation particulier de l'invention représentant le dispositif à l'état fermé du capot,
- la figure 2 est une vue schématique en coupe du dispositif de la figure 1 avec le capot en position d'utilisation,
- la figure 3 est une vue schématique en perspective du dispositif selon une forme de réalisation particulière de l'invention, avec le capot en position d'utilisation, et
- les figures 4a et 4b montrent schématiquement deux variantes d'utilisation du dispositif de l'invention.

La présence invention s'applique à tout dispositif de pulvérisation ou de distribution d'un produit fluide ou pulvérulent comportant un organe de distribution tel qu'une pompe ou une valve doseuse, associé à un réservoir contenant le produit, ledit organe de distribution comportant un orifice de distribution. Le dispositif comporte en outre des moyens d'actionnement dudit organe de distribution pour délivrer une dose de produit, et, selon l'invention, des moyens jauge de distance qui, dans leur position d'utilisation, sont disposés de telle façon à déterminer à chaque utilisation du dispositif une distance fixe prédéterminée entre l'orifice de distribution et la surface à traiter et/ou un organe d'entrée.

Par surface à traiter, on entend toute partie quelconque, notamment du corps humain, susceptible de recevoir le produit pulvérisé, tel que notamment des zones de la peau, la bouche, le nez, les yeux ou les poumons de l'utilisateur. Par organe d'entrée, on entend toute ouverture du corps humain sur laquelle viennent s'appuyer les moyens jauge de distance lorsque le produit est destiné à une partie interne du corps. Ainsi, par exemple, lorsque la surface à traiter sont les poumons, c'est la bouche qui agit en tant qu'organe d'entrée

Bien que l'application de l'invention soit générale, celle-ci sera décrite ci-après en référence à un mode de réalisation particulier dans lequel l'invention représente un perfectionnement du dispositif de pulvérisation divulgué dans le document FR-2 682 305. Ce document FR-2 682 305 est donc incorporé dans la présente invention à titre de référence en ce qui concerne en particulier le fonctionnement général du dispositif, à savoir notamment son déclenchement indépendant de l'effort de l'actionnement.

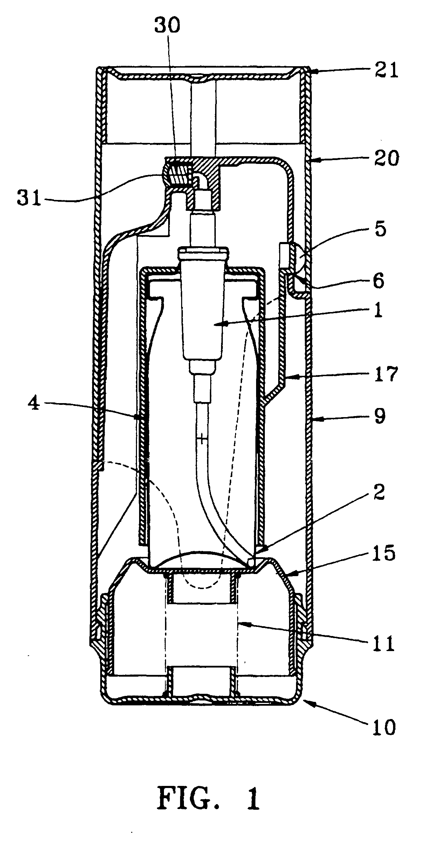

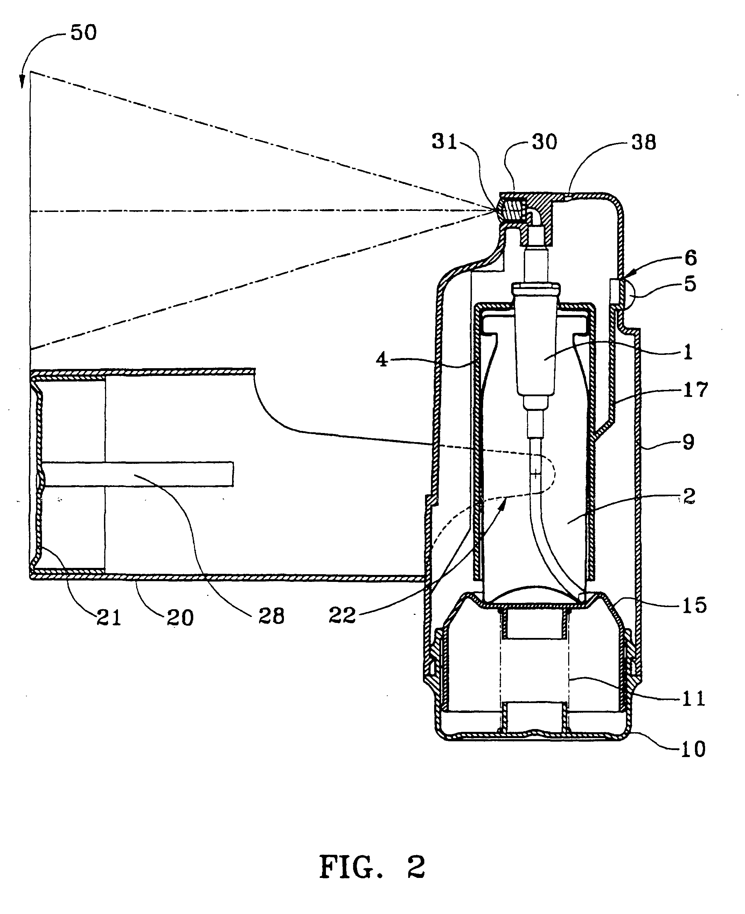

En référence aux figures 1 et 2, le dispositif comporte une pompe 1 montée sur un réservoir 2 et disposé à l'inférieur d'un étui 4, ladite pompe 1 étant mobile entre une position de repos et une position de distribution, où elle délivre une dose de produit. Pour ce faire, elle comporte des moyens d'actionnement, de préférence mécaniques, qui comportent des moyens élastiques tel qu'un ressort 11 agissant sur la pompe 1 lorsqu'elle dans sa position de repos pour la solliciter vers sa position de distribution, des moyens de verrouillage 5, 6 qui bloquent lesdits moyens élastiques 11 et maintiennent l'organe de distribution 1 c'est-à-dire la pompe 1 dans sa position de repos, et des moyens de déclenchement 5, 17 pour libérer lesdits moyens de verrouillage. Ainsi, lorsque l'utilisateur actionne lesdits moyens de déclenchement 5, 17, la pompe 1 est amenée par le ressort 11 vers sa position de distribution, où elle délivre une dose de produit à travers son orifice de distribution 31.

De préférence, le l'essort est disposé entre un embout d'extrémité inférieure 10 fixé au corps 9 de l'appareil et un élément de support mobile 15 qui supporte le récipient 2. Ainsi, lorsque les moyens de déclenchement sont actionnés par l'utilisateur, l'élément de support 15 entraîne le réservoir, l'étui 4 et la pompe 1 à se déplacer vers le haut sur les figures sous l'effet de la force du ressort 11, de sorte que la pompe est actionnée.

Comme représenté sur les figures 1 et 2, les moyens de verrouillage et de déclenchement comportent avantageusement un ergot 5 qui vient s'encliqueter dans une ouverture 6 solidaire du corps 9, l'ergot 5 étant solidaire d'une patte élastique 17 fixée audit étui 4 entourant le réservoir 2 et la pompe 1. Ledit ergot 5 est donc sollicité par la patte élastique 17 vers sa position encliquetée où il verrouille le ressort 11.

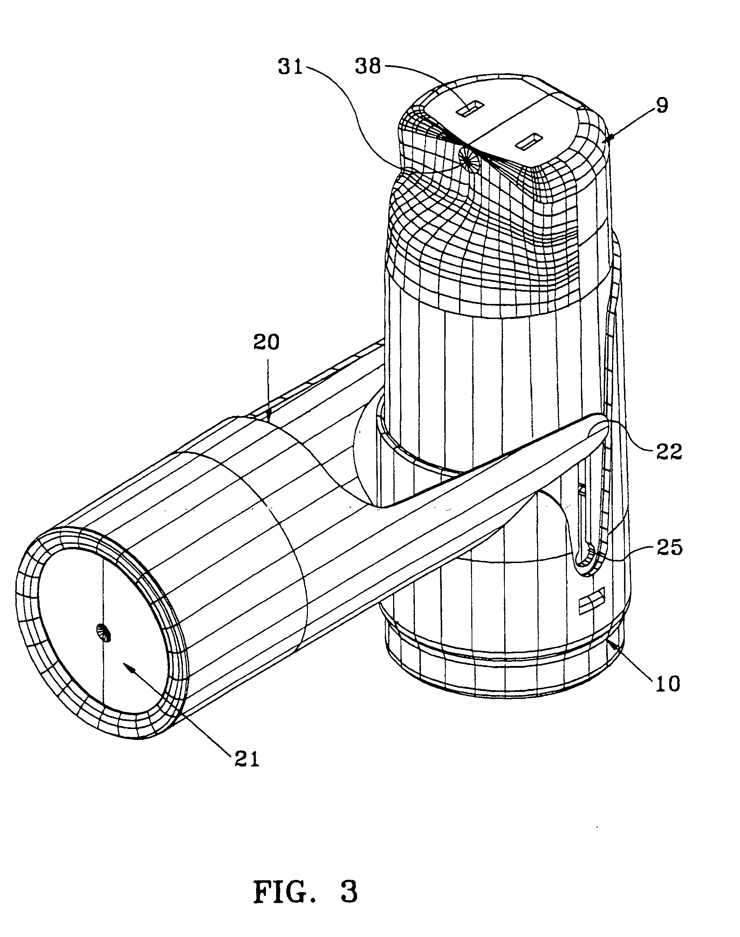

Selon l'invention, le dispositif comporte des moyens jauge de distance 20 qui en position d'utilisation permettent de déterminer et de fixer la distance entre la zone à traiter et ou l'organe d'entrée 50 et l'orifice de distribution 31. De préférence comme représenté sur les figures, les moyens jauge de distance 20 sont formés par le fourreau 20 pourvu de son capot 21 divulgué dans le document FR-2 682 305. Ainsi, dans leur position de repos, lesdits moyens jauge de distance obturent ledit orifice de distribution 31 de la pompe 1 pour le protéger, par exemple contre la poussière et recouvrent également avantageusement l'ergot de déclenchement 5, empêchant ainsi un actionnement non souhaité ou intempestif du distributeur. Pour pouvoir utiliser le distributeur, il faut donc retirer lesdits moyens jauge de distance 20. Pour ce faire, le fourreau 20 doit exercer avantageusement d'abord un mouvement de translation puis un mouvement de rotation. Ainsi, comme en particulier visible sur la figure 3, le fourreau 20 comporte deux paires 22 reliées en permanence au corps 9 du distributeur, lesdites pattes 22 coulissant en translation dans les rainures correspondances 25 ménagées dans le corps 9, de sorte que lorsque l'on souhaite utiliser l'appareil, on soulève d'abord le fourreau 20 en translation dans une direction parallèle à l'axe central du distributeur puis on le fait pivoter, avantageusement de 90°, autour d'un axe transversal audit axe central du distributeur. On arrive alors dans la position représentée sur les figures 2 et 3. De même, après utilisation du distributeur, on pivote avantageusement d'abord le fourreau 20 autour de son axe de rotation, puis on appuie dans une direction parallèle à l'axe central du distributeur pour à nouveau faire coulisser les pattes 22 dans les rainures 25. Pendant cette translation, des moyens de réarmement 28 prévus dans le fourreau 20, traversant une ou plusieurs ouverture(s) 38 dans le corps 9 et coopèrent avec l'étui 4 pour déplacer celui-ci vers la position de repos de la pompe 1 contre la force du ressort 11. Lorsque la pompe atteint à nouveau sa position de repos, le ressort 11 est comprimé et l'ergot 5 sous l'effet de l'élasticité de la patte élastique 17 vient s'encliqueter dans l'ouverture 6 du corps 9 pour verrouiller la pompe dans sa position de repos. L'appareil est alors prêt pour une utilisation ultérieure.

Il doit être entendu que l'invention ne se limite pas au mode de réalisation décrit ci-dessus et qu'au contraire l'invention s'applique à tout type de distributeur comportant une pompe précontrainte et des moyens jauge de distance assurant aussi le réarmement de la pompe. Ces moyens jauge de distance peuvent être déplacés par rapport à l'appareil d'une quelconque manière appropriée à condition qu'il permette de définir, lors de l'utilisation du dispositif, une surface à traiter reproductible en garantissant à chaque fois la même distance entre l'orifice de distribution et la surface à traiter et/ou l'organe d'entrée. De plus, selon l'invention, lesdits moyens jauge de distance assurent également, lorsqu'ils sont ramenés dans leur position de repos, le réarmement de la pompe ou de son dispositif d'actionnement, comme décrit ci-dessus, en référence à un exemple avantageux de réalisation.

De préférence, le dispositif comporte en outre un gicleur 30 disposé à proximité de l'orifice de distribution 31, et destiné à configurer de manière reproductible la pulvérisation de la dose de produit. Ainsi, en combinaison avec une distance de pulvérisation fixe, le dispositif de l'invention permet d'obtenir également une répartition fixe et reproductible de la dose de produit sur la surface à traiter et/ou l'organe d'encrée.

L'appareil représenté schématiquement sur les figures 1, 2 et 3 est particulièrement destiné à être utilisé dans une utilisation transdermale, ou un produit approprié doit être distribué sur une surface à traiter correspondant à une partie de la peau du patient. Dans cette utilisation, les moyens jauge de distance 20 viennent s'appuyer sur le corps du patient à proximité de ladite surface à traiter 50 pour déterminer de manière précise la distance entre l'orifice de distribution 31 et ladite surface à traiter, et ainsi obtenir une surface à traiter 50 reproductible de manière prévue. Le produit peut dans ce cas être une thérapie hormonale substitutive, par exemple constituées d'oestrogènes.

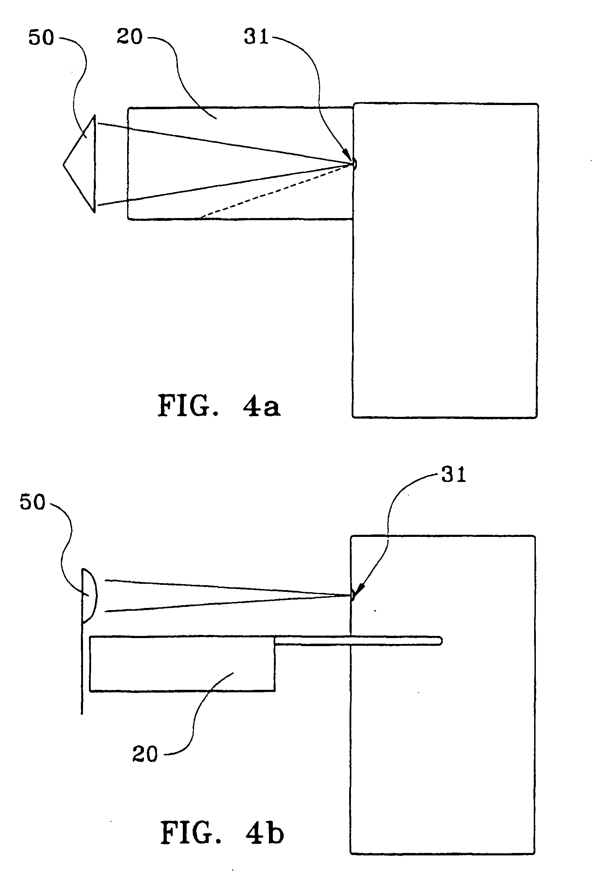

Les figures 4a et 4b représentent très schématiquement deux autres utilisations possibles du dispositif de l'invention. Ainsi, en référence à la figure 4b, le dispositif peut ètre utilisé dans le domaine de l'ophtalmologie pour distribuer un produit liquide dans un oeil 50. Dans ce cas, lesdits moyens jauge de distance 20 viennent de préférence s'appuyer sous ou autour dudit oeil 50.

Sur la figure 4a, le dispositif de l'invention est utilisé pour distribuer un produit liquide ou pulvérulent dans les poumons de l'utilisateur. Dans ce cas, les moyens jauge de distance 20 viennent s'appuyer sur l'organe d'entrée, c'est-à-dire la bouche 50 de l'utilisateur. Ainsi, dans ce mode d'utilisation, le produit est distribué à l'intérieur desdits moyens jauge de distance 20, la longueur desdits moyens jauge de distance étant choisie de telle sorte que seules les particules de produit finement pulvérisées atteignent la bouche 50 ce qui permet d'éliminer les plus grosses particules qui, elles, tombent sous l'effet de leur poids à l'intérieur desdits moyens jauge de distance 20 avant d'atteindre la bouche 50. Ceci est représenté sur la figure 4a par la ligne pointillée.

Bien entendu, des variantes de réalisation du dispositif de l'invention peuvent être utilisées à condition qu'elles remplissent les mêmes fonctions susmentionnées dans chaque cas d'utilisation.

Le dispositif selon l'invention permet donc de remplir de manière optimale les exigences susmentionnées. Ainsi, lorsque le dispositif est utilisé en ophtalmologie, la jauge de distance, qui se place sous ou autour de l'oeil à traiter, garantit le bon fonctionnement de l'appareil et assure que la goutte atteindra bien l'oeil, favorisant ainsi l'observance. D'autre part, dans le cas d'une utilisation transdermale, la jauge de distance permet d'optimiser la biodisponibilité en positionnant le dispositif toujours à distance égale de la peau, de sorte qu'on obtient une surface traitée homogène et reproductible. En outre, dans le cas d'utilisation pulmonaire, la jauge de distance permet notamment de réduire les effets secondaires en permettant de piéger les grosses particules et de ne laisser échapper que les petites.

## Revendications

1. Dispositif de pulvérisation ou de distribution d'un produit fluide ou pulvérulent sur une surface à traiter et/ou un organe d'entrée (50), comportant :
- un organe de distribution (1), comportant un orifice de distribution (31), ledit organe de distribution étant associé à un réservoir (2) contenant ledit produit et étant mobile entre une position de repos et une position de distribution, et
- des moyens d'actionnement (5, 6, 11, 17) pour déplacer ledit organe de distribution (1) vers sa position de distribution pour délivrer une dose de produit,
**caractérisé en ce que** le dispositif comporte en outre
- des moyens jauge de distance (20) déplaçables entre une position de repos, dans laquelle ils empêchent l'actionnement dudit organe de distribution (1), et une position d'utilisation, dans laquelle lesdits moyens jauge de distance s'étendent dans une direction parallèle à la direction de distribution du produit fluide ou pulvérulent, de telle sorte que les moyens jauge de distance peuvent s'appuyer sur et/ou autour de la surface à traiter et/ou de l'organe d'entrée (50), de telle sorte que l'orifice de distribution (31) est situé à une distance fixe prédéterminée de ladite surface à traiter et/ou dudit organe d'entrée (50), le déplacement desdits moyens jauge de distance (20) de leur position d'utilisation vers leur position de repos, après actionnement de l'organe de distribution (1), ramenant ledit organe de distribution (1) de sa position de distribution vers sa position de repos.

2. Dispositif selon la revendication 1, dans lequel lesdits moyens d'actionnement comportent des moyens élastiques (11), agissant sur l'organe de distribution (1), lorsqu'il est en position de repos, pour le solliciter vers une position de distribution où il délivre une dose complète de produit, des moyens de verrouillage (5, 6) desdits moyens élastiques pour maintenir l'organe de distribution dans sa position de repos et des moyens de déclenchement (5, 17) actionnables par l'utilisateur pour libérer lesdits moyens de verrouillage, et ainsi amener l'organe de distribution vers sa position de distribution.

3. Dispositif selon la revendication 2, dans lequel lesdits moyens jauge de distance (20) comportent des moyens de réarmement (28) qui, lorsque les moyens jauge de distance (20) sont ramenés de leur position d'utilisation vers leur position de repos, agissent sur l'organe de distribution (1) pour le ramener vers sa propre position de repos, contre la force des moyens élastiques (11), après quoi ces moyens élastiques (11) sont verrouillés par lesdits moyens de verrouillage (5, 6).

4. Dispositif selon la revendication 3, dans lequel lesdits moyens jauge de distance (20) sont réalisés sous la forme d'un fourreau (20) comportant un capot (21), ledit capot (21),en position de repos, obturant l'orifice de distribution (31) de l'organe de distribution (1) et ledit fourreau (20), en position de repos, recouvrant lesdits moyens de déclenchement (5), empêchant ainsi l'actionnement du dispositif.

5. Dispositif selon la revendication 3 ou 4, dans lequel lesdits moyens jauge de distance (20) sont en permanence reliés audit dispositif, le déplacement desdits moyens jauge de distance (20) entre la position de repos et leur position d'utilisation étant constitué d'une translation parallèle à l'axe central du dispositif et d'une rotation autour d'un axe perpendiculaire à l'axe central du dispositif, lesdits moyens de réarmement (28) agissant pendant ledit mouvement de translation lorsque les moyens jauge de distance (20) sont ramenés vers leur position de repos.

6. Dispositif selon la revendication 5, dans lequel ledit dispositif comporte un corps (9), lesdits moyens jauge de distance (20) comportant deux pattes (22) reliées au corps (9) du dispositif, lesdites pattes (22) coulissant dans deux rainures (25) ménagées dans ledit corps (9), lesdites deux pattes (22) pouvant en outre pivoter d'environ 90° autour d'un axe de rotation disposé transversalement à l'axe central du dispositif au niveau des extrémités supérieures des deux rainures (25).

7. Dispositif selon l'une quelconque des revendications 1 à 6, comportant en amont dudit orifice de distribution (31), un gicleur (30) permettant de définir la configuration spatiale de la pulvérisation de la dose de produit.

8. Dispositif selon l'une quelconque des revendications précédentes, dans lequel les moyens jauge de distance sont adaptés à coopérer avec une partie du corps humain.

9. Dispositif selon l'une quelconque des revendications précédentes, dans lequel l'organe de distribution (1) est une pompe.

10. Dispositif selon l'une quelconque des revendications 1 à 8, dans lequel l'organe de distribution (1) est une valve doseuse.

11. Dispositif selon l'une quelconque des revendications 2 à 10, dans lequel les moyens élastiques (11) sont réalisés sous la forme d'un ressort.

12. Dispositif selon l'une quelconque des revendications précédentes, dans lequel le dispositif de distribution est un distributeur de poudre.

13. Dispositif selon l'une quelconque des revendications 1 à 11, dans lequel le dispositif de distribution est un distributeur de liquide, lesdits moyens jauge de distance (20) étant adaptés à s'appuyer sous ou autour d'un oeil (50).

14. Dispositif selon l'une quelconque des revendications 1 à 12, dans lequel lesdits moyens jauge de distance (20) sont adaptés à s'appuyer sur ou autour de la bouche (50) de l'utilisateur, le produit étant distribué à l'intérieur desdits moyens jauge de distance (20), la longueur desdits moyens jauge de distance (20) étant choisie de telle sorte que seules les particules de produit finement pulvérisées atteignent la bouche (50), les particules plus grosses tombant sous l'effet de leur poids dans lesdits moyens jauge de distance (20) avant d'atteindre la bouche.

## Patentansprüche

1. Zerstäubungs- oder Abgabevorrichtung für ein fluid- oder pulverförmiges Produkt auf eine zu behandelnde Oberfläche und/oder ein Einlaßorgan (50), die folgende Bestandteile umfasst:
- ein Abgabeorgan (1), das eine Abgabeöffnung (31) aufweist, wobei das Abgabeorgan einem das Produkt enthaltenen Behälter (2) zugeordnet und zwischen einer Ruhelage und einer Abgabestellung bewegbar ist, und
- Betätigungseinrichtungen (5, 6, 11, 17) zum Verschieben des Abgabeorgans (1) zu seiner Abgabestellung, um eine Dosis des Produktes abzugeben,
**dadurch gekennzeichnet, daß** die Vorrichtung weiterhin umfasst:
- Abstandslehreneinrichtungen (20), die zwischen einer Ruhelage, in der sie die Betätigung des Abgabeorgans (1) verhindern, und einer Verwendungsposition verschiebbar sind, in der die Abstandslehreneinrichtungen sich in einer zur Abgaberichtung des fluid- oder pulverförmigen Produktes parallelen Richtung derart erstrecken, daß sich die Abstandslehreneinrichtungen auf und/oder um die zu behandelnde Oberfläche herum und/oder um das Einlaßorgan (50) herum derart abstützen können, daß die Abgabeöffnung (31) mit einem festen, vorbestimmten Abstand von der zu behandelnden Oberfläche und/oder dem Einlaßorgan (50) positioniert ist, wobei eine Verschiebung dieser Abstandslehreneinrichtungen (20) aus ihrer Verwendungsstellung zu ihrer Ruhelage nach einer Betätigung des Abgabeorgans (1) das Abgabeorgan (1) aus seiner Abgabestellung zu seiner Ruhelage zurückbewegen.

2. Vorrichtung nach Anspruch 1, bei der die Betätigungseinrichtungen elastische Einrichtungen (11) umfassen, die auf das Abgabeorgan (1) einwirken, wenn es sich in seiner Ruhelage befindet, um es zu einer Abgabestellung hin vorzuspannen, in der es eine vollständige Dosis des Produktes abgibt, sowie Verriegelungseinrichtungen (5, 6) für die elastischen Einrichtungen, um das Abgabeorgan in seiner Ruhelage zu halten, sowie Auslöseeinrichtungen (5, 17), die vom Verwender betätigbar sind, um die Verriegelungseinrichtungen freizugeben und so das Abgabeorgan zu seiner Abgabestellung hin zu bewegen.

3. Vorrichtung nach Anspruch 2, bei der die Abstandslehreneinrichtungen (20) Spanneinrichtungen (28) umfassen, die, wenn die Abstandslehreneinrichtungen (20) aus ihrer Verwendungsstellung zu ihrer Ruhelage zurückbewegt werden, auf das Abgabeorgan (1) einwirken, um es gegen die Kraft der elastischen Einrichtungen (11) in seine eigene Ruhelage zurückzubewegen, wonach diese, elastischen Einrichtungen (11) von den Verriegelungseinrichtungen (5, 6) verriegelt werden.

4. Vorrichtung nach Anspruch 3, bei der die Abstandslehreneinrichtungen (20) in Form einer Hülse (20) ausgebildet sind, die eine Kappe (21) umfasst, wobei die Kappe (21) in der Ruhelage die Abgabeöffnung (31) des Abgabeorgans (1) verdeckt und die Hülse (20) in der Ruhelage die Auslöseeinrichtungen (5) abdeckt und somit eine Betätigung der Vorrichtung verhindert.

5. Vorrichtung nach Anspruch 3 oder 4, bei der die Abstandslehreneinrichtungen (20) permanent mit der Vorrichtung verbunden sind, daß die Verschiebung der Abstandslehreneinrichtungen (20) zwischen der Ruhelage und ihrer Verwendungsstellung von einer Translationsbewegung parallel zur Zentralachse der Vorrichtung und einer Drehung um eine zur Zentralachse der Vorrichtung senkrechte Achse gebildet werden, wobei die Spanneinrichtungen (28) während der Translationsbewegung wirken, wenn die Abstandslehreneinrichtungen (20) in ihre Ruhelage zurückbewegt werden.

6. Vorrichtung nach Anspruch 5, bei der die Vorrichtung einen Körper (9) umfasst und die Abstandslehreneinrichtungen (20) zwei Ansätze (22) umfassen, die mit dem Körper der Vorrichtung (9) verbunden sind, wobei die Ansätze (22) in zwei Rillen (25) gleiten, die in dem Körper (9) vorgesehen sind, und die beiden Ansätze (22) weiterhin um ungefähr 90° um eine Drehachse schwenken können, die quer zur Zentralachse der Vorrichtung in Höhe des oberen Endes der beiden Rillen (25) vorgesehen ist.

7. Vorrichtung nach einem der Ansprüche 1 bis 6, die stromaufwärts von der Abgabeöffnung (31) eine Zerstäuberdüse (30) aufweist, die es ermöglicht, die Raumkonfiguration der Zerstäubung der Dosis des Produktes festzulegen.

8. Vorrichtung nach einem der vorhergehenden Ansprüche, bei der die Abstandslehreneinrichtungen ausgebildet sind, mit einem Teil des menschlichen Körpers zusammenzuwirken.

9. Vorrichtung nach einem der vorhergehenden Ansprüche, bei der das Abgabeorgan (1) eine Pumpe ist.

10. Vorrichtung nach einem der Ansprüche 1 bis 8, bei der das Abgabeorgan (1) ein Dosierventil ist.

11. Vorrichtung nach einem der Ansprüche 2 bis 10, bei der die elastischen Einrichtungen (11) in Form einer Feder ausgebildet sind.

12. Vorrichtung nach einem der vorhergehenden Ansprüche, bei der die Abgabevorrichtung eine Pulver-Abgabevorrichtung ist.

13. Vorrichtung nach einem der Ansprüche 1 bis 11, bei der die Abgabevorrichtung eine Abgabevorrichtung für eine Flüssigkeit ist, wobei die Abstandslehreneinrichtungen (20) so ausgebildet sind, daß sie sich um ein Auge (50) herum abstützen.

14. Vorrichtung nach einem der Ansprüche 1 bis 12, bei der die Abstandslehreneinrichtungen (20) geeignet sind, sich auf dem oder um den Mund (50) des Verwenders herum abzustützen, wobei das Produkt im Inneren der Abstandslehreneinrichtungen (20) abgegeben wird und die Länge der Abstandslehreneinrichtungen (20) derart gewählt ist, daß lediglich feinzerstäubte Teilchen des Produktes den Mund (50) erreichen, während größere Teilchen unter der Wirkung ihres Gewichtes in die Abstandslehreneinrichtungen (20) fallen, bevor sie den Mund erreichen.

## Claims

1. A device for spraying or dispensing a fluid or a powder onto a surface to be treated and/or into an organ presenting an entrance (50), the device comprising:
• a dispenser member (1) having a dispenser orifice (31), said dispenser member being associated with a reservoir (2) containing the substance to be dispensed and being movable between a rest position and a dispensing position; and
• actuator means (5, 6, 11, 17) for moving said dispenser member (1) towards its dispensing position to dispense a dose of substance;
the device being **characterized in that** it further comprises:
• distance-gauging means (20) movable between a rest position, wherein they prevent actuation of said dispenser member (1), and an in-use position, wherein said distance-gauging means extend in a direction parallel to the dispensing direction of the fluid or powder, such that the distance-gauging means can press on and/or around the surface to be treated and/or the organ presenting an entrance (50) such that the dispenser orifice (31) is situated at a predetermined fixed distance from said surface to be treated and/or from said organ presenting an entrance (50), the act of moving said distance-gauging means (20) from their in-use position to their rest position, after actuation of the dispenser member (1), causing said dispenser member (1) to be returned from its dispensing position to its rest position.

2. A device according to claim 1, wherein said actuator means comprise resilient means (11) acting on the dispenser member (1) while it is in the rest position, urging it towards the dispensing position in which it dispenses a measured quantity of the substance in full, locking means (5, 6) for locking said resilient means to hold the dispenser member in its rest position, and trigger means (5, 17) actuatable by the user to release said locking means and thereby bring the dispenser member towards its dispensing position.

3. A device according to claim 2, wherein said distance-gauging means (20) include re-cocking means (28) which, when said distance-gauging means (20) are returned from the in-use position to the rest position, act on the dispenser member (1) to return it to its own rest position against the force of the resilient means (11), after which said resilient means (11) are locked by said locking means (5, 6).

4. A device according to claim 3, wherein said distance-gauging means (20) are implemented in the form of a sheath (20) including a cap (21), said cap (21), in the rest position, closing the dispenser orifice (31) of the dispenser member (1) and said sheath (20), in the rest position, covering said trigger means (5), thereby preventing the device from being actuated.

5. A device according to claim 3 or 4, wherein said distance-gauge means (20) are permanently connected to said device, movement of said distance-gauging means (20) between the rest position and the in-use position being constituted by a translation movement parallel to the central axis of the device and a rotation movement about an axis perpendicular to the central axis of the device, said re-cocking means (28) acting during said translation movement while the distance-gauging means (20) are being returned to the rest position.

6. A device according to claim 5, wherein said device comprises a body (9), said distance-gauging means (20) including two arms (22) connected to the body (9) of the device, said arms (22) sliding in two grooves (25) provided in said body (9), said two arms (22) also being capable of pivoting through about 90° about an axis of rotation extending transversely to the central axis of the device level with the top ends of the two grooves (25).

7. A device according to any one of claims 1 to 6, including a nozzle (30) upstream from said dispenser orifice (31), thereby defining the spatial configuration with which the dose of substance is sprayed.

8. A device according to any preceding claim, wherein the distance-gauging means can co-operate with a portion of the human body.

9. A device according to any preceding claim, wherein the dispenser member (1) is a pump.

10. A device according to any one of claims 1 to 8, wherein the dispenser member (1) is a metering valve.

11. A device according to one of claims 2 to 10, wherein the resilient means (11) are implemented in the form of a spring.

12. A device according to any preceding claim, wherein the dispenser device is a powder dispenser.

13. A device according to any one of claims 1 to 11, wherein the dispensing device is a liquid dispenser, said distance-gauging means (20) being adapted to press beneath or around an eye (50).

14. A device according to any one of claims 1 to 12, wherein said distance-gauging means (20) are adapted to press on or around the mouth (50) of the user, the substance being dispensed inside said distance-gauging means (20), the length of said distance-gauging means (20) being selected in such a manner that only finely-divided particles of substance reach the mouth (50), with larger particles falling under the effect of their own weight inside said distance-gauging means (20) before reaching the mouth.
